# EUROPÄISCHE PATENTSCHRIFT

(11) **EP 0 775 109 B1**
(45) Veröffentlichungstag und Bekanntmachung des Hinweises auf die Patenterteilung: **20.01.1999**
(21) Anmeldenummer: 95929050.3
(22) Anmeldetag: 02.08.1995
(51) Int. Cl.: C07C 319/28, C07C 319/20, C07C 323/60, A23K 1/16

(54) **VERFAHREN ZUR HERSTELLUNG VON 2-HYDROXY-4-METHYLTHIOBUTTERSÄURE (MHA) UND DEREN VERWENDUNG ALS TIERFUTTERMITTELSUPPLEMENT**
PROCESS FOR PRODUCING 2-HYDROXY-4-METHYLTHIOBUTYRIC ACID (MHA) AND ITS USE AS FEEDSTUFF SUPPLEMENT
PROCEDE DE PRODUCTION D'ACIDE 2-HYDROXY-4-METHYLTHIOBUTYRIQUE (MHA) ET SON UTILISATION COMME COMPLEMENT D'ALIMENTS POUR ANIMAUX

(30) Priorität: 12.08.1994 DE 4428608
(43) Veröffentlichungstag der Anmeldung: 28.05.1997
(73) Patentinhaber: Degussa Aktiengesellschaft, 60311 Frankfurt (DE)
(72) Erfinder: SUCHSLAND, Helmut, D-63517 Rodenbach (DE); HÄFNER, Volker, D-63505 Langenselbold (DE)
(86) Internationale Anmeldenummer: EP9503068
(87) Internationale Veröffentlichungsnummer: WO9605173

(56) Entgegenhaltungen:
- EP-A- 0 142 488
- EP-A- 0 330 527

## Beschreibung

Die Erfindung betrifft ein Verfahren zur Herstellung von 2-Hydroxy-4-methylthiobuttersäure (MHA) nach dem Oberbegriff des Anspruchs 1.

Insbesondere betrifft die Erfindung ein verbessertes, neuartiges Verfahren zur gleichzeitigen Gewinnung von MHA und kristallinem Ammoniumsulfat oder Ammoniumbisulfat in hoher Ausbeute und Reinheit unter Vermeidung eines behandlungsbedürftigen, ggf. salzhaltigen Abwassers.

2-Hydroxy-4-methylthiobuttersäure (MHA) ist das Hydroxyanaloge der essentiellen Aminosäure Methionin in racemischer Form und ist wie diese ein wichtiger Zusatzstoff in der Tierernährung. In der Geflügelaufzucht zeigt MHA ähnliche wachstumsstimulierende Eigenschaften wie die dafür bekannte Aminosäure. Aber auch in anderen Bereichen der Tierernährung findet das Additiv zunehmendes Interesse.

Eingesetzt wird MHA meist in Form wässriger Konzentrate, wobei diese neben dem Monomeren noch einen gewissen Anteil an Oligomeren, hauptsächlich die di- und trimeren linearen Estersäuren enthalten. Der Gehalt an diesen Oligomeren hängt von den Herstellungsbedingungen und der gewählten Konzentration ab. Wegen ihres geringeren nutritiven Wirkungsgrades und des ungünstigen Einflusses auf die Fließeigenschaften infolge Viskositätserhöhung ist es jedoch wünschenswert, ihren prozentualen Anteil möglichst niedrig zu halten. Handelsübliche Formulierungen weisen bei einer Gesamtkonzentration von 88 - 90 Gew.-% weniger als 20 Gew.-%, vorzugsweise weniger als 17 Gew.-% in der Summe an Oligomeren auf, entsprechend einem Monomeren/Oligomeren-Verhältnis von ∼ 4 : 1 bis 5 : 1.

Bekannt ist auch die Verwendung des Calziumsalzes und des gemischten Calziumammoniumsalzes des MHA als Tierfutterzusatz. Die Herstellung dieser Salze ist aber mit höheren Gestehungskosten verbunden. Außerdem lassen sie sich als pulverförmige Feststoffe weniger leicht in die Futtermittelformulierung einmischen als die leicht zu versprühenden wässrigen Konzentrate der freien Säure mit niedrigem Oligomerenanteil.

Der Syntheseweg zum MHA besteht aus 3 Reaktionen.

Das allgemeine Verfahren zur Herstellung von MHA geht von 3-Methylthiopropionaldehyd, auch als Methylmercaptopropionaldehyd oder MMP bezeichnet, aus, der mit Cyanwasserstoff zum 2-Hydroxy-4-methylthiobutyronitril, auch als MMP-Cyanhydrin oder MMP-CH bezeichnet, umgesetzt wird (Gleichung I).

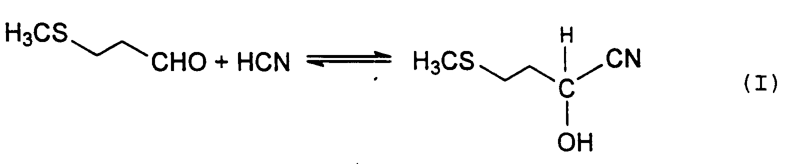

Das entstandene MMP-Cyanhydrin wird anschließend üblicherweise mit starken Mineralsäuren über die Zwischenstufe des 2-Hydroxy-4-methylthiobutyramids, auch als MHA-Amid bezeichnet (Gleichung II), 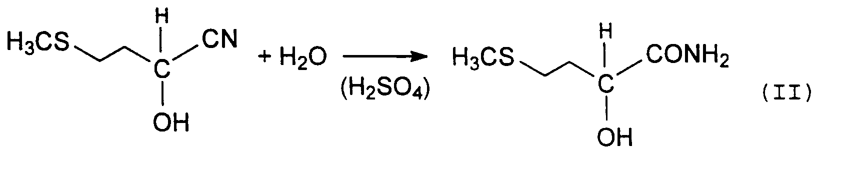 zum Methioninhydroxyanalogen (MHA) hydrolysiert (Gleichung III).

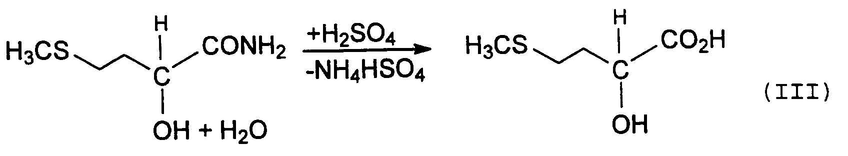

Diese Hydrolyse kann sowohl ein- als auch zweistufig durchgeführt werden.

Eine zweistufige Arbeitsweise ausgehend von MMP-Cyanhydrin beschreiben die US-Patente 2 745 745, 2 938 053 und 3 175 000. Dort wird zunächst bei relativ niedrigen Temperaturen mit konzentrierter Mineralsäure z. B. mit 50 - 85 %iger Schwefelsäure das Cyanhydrin zum MHA-Amid umgesetzt, worauf dann nach Zusatz von Wasser bei erhöhter Temperatur die Hydrolyse zum MHA weitergeführt wird. Durch Behandlung des Verseifungsgemischs mit Calziumhydroxid oder -carbonat erhält man hieraus das Calzium- bzw. Calziumammoniumsalz des MHA und als Koppelprodukt Calziumsulfat. Zur Verminderung des Zwangsanfalls wertloser Nebenprodukte empfehlen die beiden erstgenannten Patentschriften das Hydrolyseagenz Schwefelsäure im unterstöchiometrischen Verhältnis zum MMP-Cyanhydrin, beispielsweise 0,55 - 0,8 : 1 einzusetzen. Auch das britische Patent 722 024, welches die gleichartige Bildung der MHA-Salze ausgehend von MHA-Amid beschreibt, impliziert die zweistufige Arbeitsweise.

Ebenfalls der Zweistufenhydrolyse bedienen sich die in den europäischen Patentschriften 0 142 488 (mit Schwefelsäure) und 0 143 100 (mit Mineralsäure) publizierten Verfahren, die die Gewinnung von MHA in flüssiger Form, also hochkonzentrierter wässriger Lösungen, zum Gegenstand haben. Diese erhält man nach der, unter definierten Konzentrations- und Temperaturbedingungen über die Amidstufe mit überschüssiger Mineralsäure durchgeführten Hydrolysereaktion, mit Hilfe einer Lösungsmittelextraktion, wobei bestimmte, mit Wasser partiell mischbare Lösungsmittel zur Anwendung kommen. Obgleich nach diesen Verfahren MHA-Konzentrate in hoher Qualität und Ausbeute erhalten werden, besteht hier das Problem des im wässrigen Raffinat verbleibenden Koppelproduktes Ammoniumbisulfat. Über dessen Verwendung bzw. Entsorgung werden nämlich keine Angaben gemacht.

Konzentrierte MHA-Lösungen ohne Zuhilfenahme eines Lösungsmittels gewinnt man gemäß der US-Patentschrift 3 773 927 mittels Zweistufenhydrolyse des MMP-Cyanhydrins mit wässriger Salzsäure im Überschuß, nachfolgender Konzentrierung der Verseifungsmischung und Abtrennung des auskristallisierten Ammoniumchlorids. Die so erhaltenen MHA-Konzentrate sind jedoch reich an Oligomeren und schwarz verfärbt. Auch das abgetrennte Ammoniumchlorid ist stark verunreinigt.

Nach der US-Patentschrift 4 353 924 wird nach der salzsauren zweistufigen Hydrolyse der Überschuß an Mineralsäure mit Ammoniak oder anderen alkalischen Stoffe neutralisiert. Man erhält so konzentrierte MHA-Lösungen mit geringeren korrosiven Eigenschaften. Die Ammoniumsalzproblematik ist aber die gleiche. Um diese zu beseitigen wird in dem US-Patent 4 310 690 ein Verfahren beschrieben, bei welchem nach der Hydrolyse mit Salzsäure unter genau definierten Bedingungen mit Natronlauge neutralisiert und das Ammonchlorid in Kochsalz und Ammoniak umgewandelt wird. Bei der anschließenden Behandlung mit Ätzkalk erhält man das MHA-Calziumsalz als Aufschlämmung in einer praktisch gesättigten Kochsalzlösung. Nach der Fest-Flüssigtrennung werden die Filtrate größtenteils zur Bereitung der Ätzkalkaufschlämmungen wieder zurückgeführt. Auf diese Weise vermindert man die Abwasserbelastung und vermeidet die Coproduktion umweltbelastender Ballaststoffe. Über die Verwendung bzw. den Verbleib des als Zweitprodukt entstandenen Ammoniaks werden keine Angaben gemacht.

Einstufige Hydrolyseverfahren sind in der Patentliteratur ebenfalls beschrieben. So ist das Verfahren gemäß der britischen Patentschrift 915 193 auf die Gewinnung des MHA-Calziumsalzes gerichtet, wobei nach der Verseifung des MMP-Cyanhydrins mit verdünnter Schwefelsäure im Überschuß das gebildete MHA mittels Extraktion mit höher siedenden Ethern von der Verseifungslösung abgetrennt und durch nachfolgende Behandlung des Extraktes mit Calziumhydroxyd das MHA-Calziumsalz gewonnen wird. Die bei diesem kontinuierlichen Verfahren vorgesehene Rückführung des wässrigen Raffinats in die Verseifungsstufe führt allerdings zu einer Akkumulierung der anorganischen Begleitstoffe.

In der europäischen Patentschrift 0 330 527 wurde ein weiteres einstufiges Hydrolyseverfahren mit Schwefelsäure als Verseifungsagenz publiziert, das ohne Lösungsmittel auskommt und direkt zu konzentrierten wässrigen MHA-Lösungen führt, wobei als Coprodukt kristallines Ammoniumsulfat in verkaufsfähiger Form erhalten wird. Dieses Ziel wird erreicht, indem man das Verseifungsgemisch mit Ammoniumhydroxidlösung soweit neutralisiert, daß die überschüssige Mineralsäure und das entstandene Ammoniumbisulfat in das neutrale Sulfat überführt werden, wobei zwei flüssige Phasen entstehen, die ihrerseits getrennt und eingedampft werden, um flüssiges MHA einerseits und kristallines Ammoniumsulfat andererseits zu gewinnen. Dabei werden die verschiedenen Filtrations- und Rückführungsschritte so kombiniert, daß praktisch kein Produkt verloren geht und kein mit Salz belastetes Abwasser entsteht. Das resultierende MHA ist von ähnlicher Qualität wie das nach der EP 0 142 488 erhaltene Produkt.

Jedoch auch dieses umweltschonende Verfahren weist diverse Nachteile auf. Wie die Anmelderin der vorliegenden Erfindung beim Nacharbeiten dieses Verfahrens feststellte, müssen zum einen, bedingt durch die vergleichsweise höhere Verdünnung der Schwefelsäure (20 - 50 %), deutlich höhere Säureüberschüsse als angegeben verwendet werden, um zu einem vollständigen Cyanhydrin-Umsatz zu kommen. Auch muß zur Vermeidung von Salzausscheidungen während der Neutralisation in höherer Verdünnung gearbeitet werden, um die beiden flüssigen Phasen sauber trennen zu können. Zum anderen ist das isolierte Ammoniumsulfat von klebriger Konsistenz und mit einem intensiven Geruch behaftet, so daß eine Nachbehandlung wie z. B. eine Waschfiltration oder Umkristallisation unumgänglich erscheint, wodurch das Verfahren zusätzlich verteuert wird. Auch ist das Verfahren in den Eindampfschritten - anders als postuliert - energieaufwendiger als das zum Vergleich herangezogene Verfahren der EP-A 0 142 488. Kostenintensiv und apparativ sehr aufwendig ist außerdem das mit zwei getrennten Strängen ausgestattete Feststoffhandling mit Filtration/Zentrifugation sowie der im Fließdiagramm nicht angegebenen Trocknung des Ammoniumsulfats.

Angesichts des hierin angegebenen Stands der Technik sowie den mit den bekannten Verfahren verbundenen Nachteilen ist es Aufgabe der Erfindung, ein weiteres Verfahren zur Herstellung von 2-Hydroxy-4-methylthiobuttersäure (MHA) gemäß der eingangs erwähnten Gattung anzugeben, welches hinsichtlich der Aufarbeitung der Reaktionsprodukte möglichst einfach und kostengünstig sein soll aber gleichzeitig das Auftreten von unerwünschten Abfallstoffen weitgehend vermeidet.

Gelöst werden diese sowie weitere nicht im einzelnen angegebene Aufgaben mit einem Verfahren, das die Merkmale des kennzeichnenden Teils des Anspruches 1 aufweist.

Dadurch, daß bei der Isolierung des MHA's das Reaktionsgemisch unter Erhalt eines geringen Restwassergehalt aufweisenden bis praktisch Restwasserfeien MHA-haltigen Salzrückstandes eingedampft wird, der MHA-haltige Salzrückstand anschließend mit einem organischen Lösungsmittel unter Erhalt einer Suspension behandelt wird, anschließend die festen Bestandteile aus der Suspension unter Erhalt einer MHA-haltigen Lösung abgetrennt werden, danach das organische Lösungsmittel aus der MHA-haltigen Lösung unter Erhalt eines MHA-Rückstandes entfernt wird und ggf. danach der MHA-Rückstand durch Zusatz von Wasser konditioniert wird, wird erfindungsgemäß ein Verfahren zur Verfügung gestellt, welches die Herstellung von flüssigem MHA in hervorragender Qualität gestattet und welches insbesondere die Bildung eines mit Salz belasteten Abwassers vermeidet, wobei das neben kristallinem Ammoniumsulfat oder Ammoniumbisulfat anfallende flüssige MHA sich vor allem durch einen geringen Oligomeren-Anteil und durch einen hohen Reinheitsgrad auszeichnet. Insbesondere ist das erfindungsgemäß erhältliche flüssige MHA im wesentlichen frei von organischen Verunreinigungen.

Im Rahmen der Erfindung wird das MHA aus dem Reaktionsgemisch bevorzugt durch eine Fest/Flüssig-Trennung isoliert, bei der ein im wesentlichen fester MHA-haltiger Salz-Rückstand mit einem organischen, mit Wasser vollständig, teilweise oder auch nicht mischbaren inerten Lösemittel behandelt wird.

Diese Vorgehensweise unterscheidet sich deutlich von den aus dem Stand der Technik bekannten Verfahren, bei denen das MHA aus dem Reaktionsgemisch (Hydrolysat) entweder durch Flüssig/Flüssig-Extraktion (EP 0 142 488) oder durch kombinierte Flüssig/Flüssig- und Fest/Flüssig-Phasentrennung (EP 0 330 527) isoliert wird. Während die MHA-Isolierung gemäß der erstgenannten Patentschrift ohne Feststoffhandling auskommt und gemäß der letztgenannten Patentschrift die Isolierung von MHA aus einem MHA-haltigen Hydrolysat ohne Verwendung eines Lösungsmittels vonstatten geht, umfaßt die Verfahrensweise bei der Isolierung des MHA aus dem Hydrolysat gemäß der vorliegenden Erfindung zwar sowohl ein Feststoffhandling als auch die Verwendung eines organischen Lösungsmittels zur Isolierung des MHA, bietet jedoch im Gegensatz zu den aus dem Stand der Technik bekannten Verfahren den entscheidenden Vorteil, daß es neben der Herstellung von flüssigem MHA mit günstigen Eigenschaften, insbesondere mit niedrigem Oligomeren-Anteil, auch die Herstellung eines der beiden Ammoniumsalze der Schwefelsäure (Ammoniumsulfat oder Ammoniumhydrogensulfat) in hoher Reinheit, d. h. in verkaufsfähiger Form, gestattet.

Ein wesentlicher Vorteil des neuen Verfahrens ist nämlich darin zu sehen, daß im Gegensatz zu dem Verfahren gemäß der EP 142 488 keine anorganischen Salze enthaltenden und deshalb behandlungsbedürftigen Abwässer erzeugt werden, deren Entsorgung bzw. Aufbereitung problematisch und kostenintensiv ist, während im Gegensatz zu dem Verfahren der EP 330 527 diese Aufgabe mit geringerem Energieaufwand, geringerem apparativen Aufwand und bezüglich der Gewinnung von Ammoniumsulfat oder -bisulfat als Coprodukt mit höherer Produktqualität, die eine Nachbehandlung nicht erforderlich macht, gelöst wird. Insbesondere zeigt ein Vergleich des erfindungsgemäßen Verfahrens mit dem aus der EP 330 527 bekannten verfahren, daß die für die verschiedenen Eindampfschritte aufzuwendenden Energien im letztgenannten Verfahren nahezu doppelt so hoch sind, genauer gesagt 1,8-fach so hoch, wobei schließlich die für die Nachbehandlung des Salzes gemäß der EP 330 527 zur Überführung in eine verkaufsfähige Form noch erforderliche Energie unberücksichtigt blieb.

Von besonderem Vorteil im erfindungsgemäßen Verfahren ist die Eindampfung der nach der Hydrolyse erhaltenen Reaktionsmischung in einem Schritt und die Zerlegung des weitgehend bis vollständig vom Wasser befreiten Produktgemisches mit Hilfe eines inerten Lösungsmittels, in welchem das MHA löslich und das Ammoniumsulfat oder Ammoniumbisulfat praktisch unlöslich ist, in eine das MHA gelöst enthaltende flüssige Komponente und eine das Ammoniumsalz enthaltende kristalline Komponente, welche voneinander abgetrennt werden können.

Dabei kann die Eindampfung des nach der Hydrolyse erhältlichen Reaktionsgemisches auf alle dem Fachmann bekannten Arten erfolgen.

Zur Erreichung einer möglichst vollständigen späteren Abtrennung des MHA's aus dem nach dem Eindampfen zurückbleibenden MHA-haltigen Salzrückstandes ist dabei eine möglichst hohe Wasserfreiheit des Eindampfrückstandes von Nutzen. Andererseits sind die zur Eindampfung erforderlichen Bedingungen möglichst schonend zu wählen, damit eine unnötige Schädigung des MHA-haltigen Rückstandes vermieden wird. Bevorzugt sind geringe Restwassergehalte, z. B. von ≤ 5 %(Gewichtsprozent) bezogen auf das Zielprodukt MHA. Besonders vorteilhaft wird das Reaktionsgemisch nach der Hydrolyse im wesentlichen vollständig von Wasser befreit. Dabei wird unter "im wesentlichen vollständig von Wasser befreit" nicht die absolute Abwesenheit von Wasser verstanden. Vielmehr wird ein unter den bevorzugt einzuhaltenden Bedingungen von Vakuum und Temperatur üblicher Restwassergehalt akzeptiert, der jedoch bis zur praktischen Restwasserfreiheit reichen kann.

Von besonderem Vorteil für die Erfindung kann es sein, daß die Eindampfung des Reaktionsgemisches kontinuierlich ausgeführt wird. Eine kontinuierliche Verfahrensweise ermöglicht die Anwendung sehr schonender Bedingungen, insbesondere von sehr kurzen Verweilzeiten der vom Wasser zu befreienden Reaktionsmischung im für die Eindampfung verwendeten Aggregat. Hierfür kommen alle dem Fachmann geläufigen Aggregate in Frage, wozu beispielsweise geeignete Verdampfer gehören, wie z. B. ein mit Rotor ausgestatteter Dünnschichtverdampfer u. ä.

Das einzudampfende und weitgehend von Wasser zu befreiende Reaktionsgemisch kann ggf. und bevorzugt vor dem Abziehen des Wassers, bevorzugt im Vakuum, vor und/oder während der eigentlichen Eindampfung einer adiabatischen Verdampfungskühlung unter Anlegen eines Vakuums bis auf ca. 60 °C oder weniger unterzogen werden, um eventuelle flüchtige oder geruchsintensive Bestandteile des Reaktionsgemisches zu entfernen. Dadurch wird zusätzlich erreicht, daß diese Bestandteile von der später abzuziehenden Hauptmenge des Wassers getrennt gehalten werden können.

Weiterhin ist es von Vorteil, wenn die Verseifungslösung, also das Reaktionsgemisch, nach Behandlung mit Schwefelsäure mit Ammoniak, bevorzugt gasförmig, behandelt wird und dabei entweder bis zur vollständigen Bildung von Ammoniumsulfat oder nur partiell zum Abstumpfen ggf. noch vorhandener freier Schwefelsäure neutralisiert wird. Dieser Schritt kann im Falle des angestrebtem Beiproduktes Ammoniumbisulfat auch entfallen.

In der großtechnischen Realisierung des erfindungsgemäßen Verfahrens kann es ferner bevorzugt sein, daß man die nach der Fest/flüssig-Trennung in fester Form erhaltenen Ammoniumsulfat-Salze ggf. nach vorheriger Ausdampfung von Lösungsmittelresten und Anmaischen mit Wasser einer Schwelfelsäure-Kontakt-Anlage unter Rückgewinnung von Schwefelsäure zuführt.

Ferner ist es auch möglich, daß die Eindampfung des Reaktionsgemisches ohne vorherige oder nachfolgende Neutralisation mit Ammoniak durchgeführt wird, und daß das nach der Fest/flüssig-Trennung in fester Form erhaltene Ammoniumbisulfat ggf. nach vorheriger Ausdampfung von Lösungsmittelresten und Anmaischen mit Wasser einer Schwelfelsäure-Kontaktanlage unter Rückgewinnung von Schwefelsäure zugeführt wird.

Unter den für die Stofftrennung (Aufnahme des von Wasser weitgehend bis vollständig befreiten Sumpfproduktes und Abtrennung der flüssigen von der festen Phase) in Frage kommenden Lösungsmittel gibt es eine Vielzahl, die die Bedingungen der chemischen Indifferenz und einer geringen Löslichkeit für Ammoniumsulfat und/oder Ammoniumbisulfat erfüllen. Geeignete Lösungsmittel können mit Wasser mischbar, teilweise mischbar oder auch wasserunlöslich sein. In Frage kommen z. B. Ether wie Isopropylether, Tetrahydrofuran, Dimethoxyethan, sekundäre Alkohole wie 2-Propanol, Sekundär-Butylalkohol, Ketone wie Aceton, Methylethylketon, Methylisopropylketon, Methylisobutylketon, aromatische Kohlenwasserstoffe wie Toluol, Chlorkohlenwasserstoffe wie Tetrachlorkohlenstoff und andere. Weniger geeignet sind primäre Alkohole, Ester und aliphatische und cycloaliphatische Kohlenwasserstoffe. Unter verfahrenstechnischen Gesichtspunkten haben sich als besonders geeignet polare Lösungsmittel erwiesen, die vergleichsweise niedrige Siedepunkte und Verdampfungswärme aufweisen und die sich destillativ einfach, ggf. unter rektifizierenden oder azeotropbildenden Bedingungen, zurückgewinnen lassen. Im Falle der (partiellen) Wassermischbarkeit ist außerdem zu beachten, daß der Wassergehalt bei einer möglichen Kreislaufführung des Lösungsmittels 5 Gew.-% nicht übersteigen sollte. Im Rahmen der Erfindung bevorzugte Lösungsmittel sind Aceton, Methylisopropylketon, Methylisobutylketon, Isopropanol, Tetrahydrofuran und Toluol. Besonders bevorzugt ist Aceton. Auch Mischungen der vorgenannten Lösungsmittel können erfindungsgemäß eingesetzt werden.

Bei der Erfindung wird aus einer gallertartigen Masse, die man als MHA-haltigen Salzrückstand nach der Eindampfung erhält, durch Zusatz eines erfindungsgemäßen Lösungsmittels eine Suspension, welche insbesondere kristallin ausfallendes Salz (Ammoniumsulfat oder Ammoniumbisulfat) enthält. Das durch Zugabe des Lösungsmittels in Form einer Suspension erhaltene kristallin ausgefallene Salz wird schließlich unter Erhalt einer MHA-haltigen Lösung abgetrennt. Diese Trennung kann grundsätzlich nach allen Verfahrensvarianten durchgeführt werden, die der Fachmann für die Abtrennung von Feststoffen aus Lösungen kennt. Bevorzugt eingesetzte Verfahren sind die Filtration unter Schwerkrafteinfluß oder auch die Zentrifugation. Die auskristallisierten und so abgetrennten Ammoniumsalze werden ggf. noch mit dem verwendeten Lösungsmittel ausgewaschen und anschließend getrocknet. Auf diese Weise erhältliches und behandeltes Ammoniumsulfat oder Ammoniumbisulfat ist im wesentlichen frei von organischen Verunreinigungen und besitzt bei einem Reinheitsgrad von ≥ 99 % Verkaufsqualität.

Die nach der Abtrennung der festen Bestandteile aus der Suspension als Filtrat oder Zentrifugat erhaltene MHA-haltige Lösung (organische Phase) wird gemäß der Erfindung zur Isolierung des darin enthaltenen MHA's weiter behandelt. Dies geschieht vorzugsweise durch Abdampfen des Lösungsmittels, ggf. unter rektifizierenden oder azeotropbildenden Bedingungen, wobei weiterhin bevorzugt das zurückgewonnene Lösungsmittel kein oder höchstens bis 5 Gew.-% Wasser aufweist. Hierbei ist es wiederum bevorzugt, um ggf. mögliche Schädigungen des MHA's zu vermeiden, dessen thermische Beanspruchung durch Anlegen eines Vakuums möglichst gering zu halten.

Das nach der Abdampfung des Lösungsmittels aus der MHA-haltigen Lösung erhältliche MHA ist bereits von hoher und verkaufsfähiger Qualität. Es kann jedoch optionell dem aus der Eindampfung der organischen Phase unter schonenden Bedingungen als Sumpfprodukt abfließenden MHA zur Konditionierung Wasser zugesetzt werden, um flüssiges MHA in einer gewünschten Konzentration von ca. 85 - 90 Gew.-% (einschließlich Oligomere) zu erhalten.

Weiterhin ist das erfindungsgemäße Verfahren hinsichtlich der Durchführung der Verfahrensschritte Eindampfung - Kristallisation - Filtration/Zentrifugation - Lösungsmittelrückgewinnung und Endverdünnung des flüssigen MHA wahlweise kontinuierlich oder intermittierend zu gestalten. Die kontinuierliche oder intermittierende Arbeitsweise ermöglicht eine besonders schonende thermische Produktbehandlung mit Gesamtverweilzeiten des MHA vom Ende der Hydrolyse bis ggf. Verdünnung mit Wasser von weniger als 60 Minuten, vorzugsweise von weniger als 30 Minuten. Durch diese Art der Isolierung ist weiterhin besonders vorteilhaft die Gewinnung eines ca. 85 - 95 Gew.-%igen flüssigen MHA-Produktes mit äußerst geringer Verfärbung, guter Fließeigenschaft, guter thermischer Stabilität und mit vergleichsweise geringem Oligomerenanteil von höchstens 17 Gew.-%, vorzugsweise weniger als 15 Gew.-% bezogen auf das Endprodukt gewährleistet.

In einem weiteren Aspekt verbessert das erfindungsgemäße Verfahren zusätzlich zur Isolierung des MHA's aus dem durch Hydrolyse mit Schwefelsäure erhaltenen Reaktionsgemisch auch die Hydrolyse des MMP-CH's selbst. So wird in einer erfindungsgemäß bevorzugten Verfahrensvariante die Hydrolyse des MMP-Cyanhydrins in zwei Stufen ausgeführt, wobei in einer ersten Stufe das MHA-Amid und in einer zweiten Stufe das MHA erhalten wird. Dabei wird bevorzugt die Hydrolyse von MHA-Amid in einer ersten Stufe mit 60 - 85 %iger, vorzugsweise 65 - 75 %iger Schwefelsäure im Molverhältnis 1 : 0,5 bis 1 : 1,0, vorzugsweise 1 : 0,55 bis 1 : 0,95, bei Temperaturen zwischen 20 und 60 °C, vorzugsweise 30 - 50 °C, durchgeführt. Hierbei entsteht im wesentlichen aus dem MMP-Cyanhydrin das MHA-Amid, wobei die entstehende Mischung weiterhin im wesentlichen und vorteilhaft praktisch frei von nicht umgesetztem Cyanhydrin ist.

Weiterhin ist es erfindungsgemäß bevorzugt, die Hydrolyse des in der ersten Stufe erhaltenen MHA-Amids in einer zweiten Stufe durch Zugabe von Wasser und geringstenfalls weiterer Schwefelsäure bis zu der stöchiometrischen Obergrenze bei Temperaturen von 90 - 110 °C, vorzugsweise unter Rückflußbedingungen, auszuführen, um die Hydrolyse des MHA-Amids zum MHA zu vervollständigen. Hierbei ist im Rahmen der Erfindung die Zwei-Stufen-Hydrolyse des MMP-Cyanhydrins mit im Vergleich zum stand der Technik höher konzentrierter Schwefelsäure in unterstöchiometrischem bis höchstens stöchiometrischem Verhältnis durchzuführen. Hierbei kann im Falle der unterstöchiometrischen Dosierung in der ersten Hydrolysestufe (Amidbildung) bei niedrigerer Temperatur zur Verkürzung der Reaktionsdauer in der zweiten Stufe weitere Schwefelsäure ggf. bis zum Erreichen der stöchiometrischen Obergrenze bei erhöhter Temperatur zugeführt werden, um die Umwandlung des Amids zur Säure zu vervollständigen.

Insgesamt wird mit dem erfindungsgemäßen Verfahren samt der genannten bevorzugten Ausführungsformen des Verfahrens eine Einsparung von Rohstoffen ermöglicht, d. h. von Schwefelsäure durch Einsatz unterstöchiometrischer bis höchstens gleicher Äquivalente, wobei gleichzeitig neben dem als Endprodukt angestrebten flüssigen MHA kristallines verkaufsfähiges Ammoniumbisulfat oder Ammoniumsulfat ohne Bildung eines mit Salzen belasteten Abwassers bei relativ niedrigen Energie- und Gesamtüberführungskosten gewonnen werden kann.

Weitere Aspekte der vorliegenden Erfindung ergeben sich aus den nachfolgend beschriebenen Beispielen, in denen auf die beigefügten Figuren Bezug genommen wird.

In den Figuren zeigen:
- Figur 1:: eine schematische Übersicht für eine erste technische Variante des erfindungsgemäßen Verfahrens zur Herstellung von MHA;
- Figur 2:: eine schematische Übersicht für eine zweite technische Variante des erfindungsgemäßen Verfahrens zur Herstellung von MHA;
- Figur 3:: eine schematische Übersicht für eine dritte technische Variante des erfindungsgemäßen Verfahrens zur Herstellung von MHA;

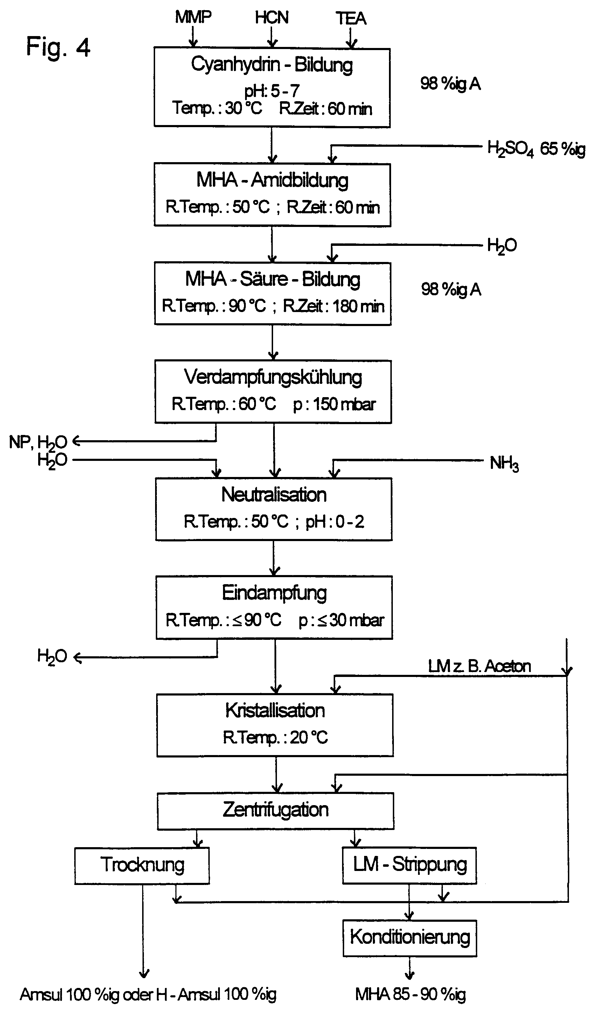

- Figur 4:: ein Blockdiagramm mit Darstellung der Verfahrensschritte einer bevorzugten erfindungsgemäßen Verfahrensvariante.

### Beispiele

### Beispiel 1:

### Herstellung von 2-Hydroxy-4-methylthiobutyronitril (MMP-CH)

In einem mit pH-Elektrode versehenen kühlbaren Reaktionsgefäß wurden 513,8 g 99,0 %iger 3-Methylthiopropionaldehyd (4,883 Mol) vorgelegt. Mit Triethylamin wurde der pH-Wert von 6,2 auf 7,5 angehoben. Danach wurden bei 30 °C ± 2 °C unter intensiver Rührung und Kühlung innerhalb 30 - 45 Minuten 135,1 g 99,9 %iger Cyanwasserstoff (4,993 Mol) eingeleitet, wobei der pH-Wert der Reaktionslösung mittels weiterer Zugabe von Triethylamin bei 7,5 konstant gehalten wurde. Bei Beendigung des Cyanwasserstoff-Zulaufs waren insgesamt 1,08 g Triethylamin verbraucht. Danach wurde die Kühlung entfernt und die Lösung noch weitere 30 Minuten bei 30 °C gerührt. Nach dem Abkühlen auf Umgebungstemperatur wurden 649,6 g einer 98,6 %igen Lösung von 2-Hydroxy-4-methylthiobutyronitril erhalten, die durch Zugabe von 85 %iger Phosphorsäure auf einen pH-Wert von 2,6 stabilisiert wurde. Die Analyse ergab eine Umsatzausbeute von 99,96 % bezogen auf den eingesetzten 3-Methylthiopropionaldehyd.

### Beispiel 2:

In einem mit Intensivrührer ausgestatteten Reaktionsgefäß wurden 150,2 g 65,3 %ige Schwefelsäure (1,0 Mol) bei 50 °C vorgelegt. Innerhalb 30 Minuten wurde bei dieser Temperatur unter intensivem Rühren und Kühlen 133,1 g 98,6 %iges MMP-CH (1,0 Mol) zugegeben. Bei unveränderter Reaktionstemperatur ließ man die Mischung weitere 30 Minuten nachreagieren, worauf mittels HPLC-Analyse die vollständige Umsetzung des Cyanhydrins zum 2-Hydroxy-4-methylthiobutyramid (MHA-Amid) festgestellt wurde. Anschließend wurde der Reaktorinhalt mit 95 g Wasser verdünnt, auf 90 °C erwärmt und bei dieser Temperatur 150 Minuten lang gerührt. Nachdem man sich von der Vollständigkeit der Hydrolyse der Amidstufe zur freien Säure MHA mittels HPLC-Analyse vergewissert hatte, wurde Vakuum angelegt und die Temperatur des Reaktionsgemischs auf ca. 70 °C unter Verdampfungskühlung abgesenkt. Dabei wurden ca. 8 g an flüchtigen Bestandteilen abgetrieben. die entgeistete Lösung wurde zur Umwandlung des bei der Reaktion gebildeten sauren Ammoniumsulfats in neutrales Ammoniumsulfat mit 1 Mol gasförmigen oder konzentriert wässrigen Ammoniak behandelt. Daraufhin wurde in einem rotierenden Schnellverdampfer unter Vakuum möglichst rasch das Wasser vollständig abgedampft und das zurückbleibende Sumpfprodukt mit 200 g Aceton aufgenommen. Hierbei ging das MHA in Lösung, während sich das unlösliche Ammoniumsulfat in kristalliner Form absetzte. Das Salz wurde abfiltriert, mit 25 g Aceton nachgewaschen und getrocknet. Man erhielt 130 g 99,95 %iges Ammoniumsulfat. Die vereinigten acetonischen Filtrate wurden in einem rotierenden Schnellverdampfer vom Lösungsmittel befreit und das resultierende Sumpfprodukt mit ca. 20 g Wasser verdünnt, so daß eine ca. 88 %ige MHA-Lösung erhalten wurde. Die Ausbeute an MHA einschließlich Oligomeren betrug 98,5 % bezogen auf eingesetztes MMP-CH. Das Produkt war von gelblicher Farbe. Es hatte einen Gehalt von 11,0 % an dimeren und weniger als 2 % an trimeren Anteilen.

### Beispiele 3 - 8:

Es wurde wie in Beispiel 2 beschrieben verfahren, jedoch wurden für die Salz-Produkttrennung anstelle des Acetons nacheinander folgende Lösungsmittel eingesetzt: Isopropanol, Methyl-t-butylether, Tetrahydrofuran, Essigester, Toluol, Methylethylketon und Methylisobutylketon. Es wurden praktisch identische Ergebnisse an MHA und Ammoniumsulfat erhalten. Der Anteil an Dimeren schwankte zwischen ca. 10 bis ca. 13 %, der Anteil an Trimeren lag unter ca. 2 %. Bei Verwendung von Tetrahydrofuran als Trennmittel wurde wegen der schlechteren Filtrierbarkeit wiederholt nachgewaschen.

### Beispiel 9:

1 Mol 98,6 %iges MMP-CH wurde wie in Beispiel 2 beschrieben mit 1 Mol Schwefelsäure zu MHA umgesetzt. Nach beendeter Reaktion wurde das Hydrolysat ohne vorherige Neutralisation mit Ammoniak unter Vakuum bis zur Gewichtskonstanz eingedampft. Das praktisch wasserfreie Sumpfprodukt wurde mit 200 g Aceton behandelt. Die resultierende Suspension wurde zentrifugiert, der Filterkuchen mit 25 g Aceton gewaschen und getrocknet. Filtratmutterlauge und Waschlösung wurden vereinigt und unter Vakuum lösungsmittelfrei destilliert. Man erhielt 148,7 g eines öligen Rückstandes entsprechend einer Gesamtausbeute von 99 % an MHA, der nach Verdünnung mit Wasser eine gelb verfärbte ca. 88 %ige MHA-Lösung mit guten Fließeigenschaften und guter thermischer Beständigkeit ergab. Der Dimerengehalt wurde mit 15,1 % ermittelt während der Trimerengehalt unter 1,5 % lag. Außerdem wurden 113 g kristallines, praktisch reines Ammoniumbisulfat in rieselfähiger Form erhalten.

### Beispiel 10:

In einer Apparatur mit Intensivrührer und Rückflußkühler wurden 80 g 98 %ige Schwefelsäure (0,8 Mol) mit 40 g Wasser verdünnt (65,3 %ig) und auf 50 °C erwärmt. In einem Zeitraum von 30 Minuten ließ man unter guter Durchmischung und Rührung 133,1 g 98,6 %iges MMP-CH (1,0 Mol) einfließen. Nach weiteren 30 Minuten bei 50 °C zeigte die HPLC-Analyse vollständige Umsetzung des MMP-CH zum Hydrolysezwischenprodukt an. Die viskose Mischung wurde mit 75 g Wasser verdünnt (40,2 %ig) und auf 100 - 102 °C erhitzt. Nach 3,5 stündigem Kochen unter Rückflußbedingungen war die Reaktion beendet und kein MHA-Amid mehr nachweisbar. Die bräunlich verfärbte Lösung wurde durch Verdampfungskühlung auf 65 °C abgekühlt. Dabei wurden ca. 15 g flüchtiger Bestandteile entfernt. Anschließend wurden 13,7 g Ammoniakgas (0,8 Mol) zur Neutralisation eingeperlt, wonach die Mischung in einem Schnellverdampfer bis zur Gewichtskonstanz eingedampft wurde. Es verblieben 260 g einer viskosen, gelartigen Reaktionsmasse, die in 200 g Aceton aufgenommen wurde. Die resultierende Suspension wurde filtriert, der Filterrückstand 2 mal mit je 25 g Aceton digeriert und getrocknet. Man erhielt 106 g 99,7 %iges rein weißes Ammoniumsulfat. Die vereinigten Filtrate wurden am Rotationsverdampfer zur Trockne eingedampft. Es wurden 151 g öliges MHA-Konzentrat erhalten, das mit Wasser auf einen Gehalt um 88 % verdünnt wurde. Der Dimerenanteil im Konzentrat betrug 13,8 %, der Trimerenanteil lag unter 1,5 %. Die Lösung hatte eine braune Farbe.

### Beispiel 11:

Beispiel 10 wurde dahingehend abgewandelt, daß 1 Mol MMP-CH mit 0,9 Mol Schwefelsäure im gleichen Konzentrationsverhältnis (1. Stufe 65,3 %ig wässrig, 2. Stufe 40,2 %ig wässrig) zur Reaktion gebracht wurde, jedoch mit den weiteren Maßgaben, daß das Hydrolysezwischenprodukt 3 Stunden unter Rückfluß (100 - 102 °C) gekocht und für die Neutralisation 0,90 Mol Ammoniak aufgewendet wurde. Nach der Produktzerlegung mit Aceton und nachfolgender Aufarbeitung wie beschrieben, wurden 150 g MHA mit einem Dimerengehalt von 16,2 % (99,9 % Gesamtausbeute) erhalten, das nach der Verdünnung auf 88 % eine bräunlich verfärbte Lösung ergab. Außerdem wurden 119 g rein weißes Ammoniumsulfat erhalten.

### Beispiel 12:

Beispiel 2 wurde wiederholt, jedoch wurde die Hydrolysierdauer in der 2. Stufe auf 1,75 Stunden verkürzt, während die Reaktionstemperatur auf 100 - 102 °C erhöht wurde. Nach der Produktzerlegung mit Aceton und Aufarbeitung wurden 151 g MHA-Konzentrat mit 11,0 % Dimerenanteil und weniger als 2 % Trimerenanteil erhalten, das nach Verdünnung auf 88 % eine braun verfärbte Lösung mit befriedigender Fließfähigkeit und thermischer Stabilität ergab. Außerdem wurden 129 g rein weißes Ammoniumsulfat mit einem Gehalt von 99,7 % isoliert.

### Beispiel 13:

Beispiel 2 wurde wiederholt, jedoch wurden zur Neutralisation 1,15 Mol Ammoniakgas verwendet und die Dauer der Vakuumeindampfung und der Acetonentgeistung auf 15 Minuten begrenzt. Die Ausbeuten an MHA und Ammoniumsulfat waren quantitativ. Das MHA-Konzentrat war 98,0 %ig mit einem Dimerenanteil von 8,7 % und enthielt noch 2,0 % Ammoniumsulfat. Trimere waren nicht mehr nachweisbar. Die auf 88 % Wirkstoffgehalt verdünnte, gelb gefärbte Lösung war von hervorragender Fließfähigkeit und thermischer Stabilität.

### Beispiel 14:

Zu 216 g 75 %iger Schwefelsäure wurden im Verlauf von 30 Minuten 3 Mol MMP-CH entsprechend einem Molverhältnis von 0,55 : 1 bei 50 °C zugegeben. Nach 30 minütiger Nachreaktionszeit war das Cyanhydrin vollständig zu dem im wesentlichen aus MHA-Amid neben wenig MHA bestehenden Hydrolysezwischenprodukt umgesetzt. Danach wurde die Mischung mit 240 g Wasser auf einen Schwefelsäuregehalt von 35,5 % ohne organische Anteile verdünnt und in 3 gleiche Teile aufgeteilt.
Teil (1) wurde auf 104 °C erhitzt und 4,5 h unter Rückflußsieden gerührt, wobei nach 2,5 h 35 ml Wasser und Leichtsieder abdestilliert wurden. Nach beendeter Reaktion wurde wie im Beispiel 9 beschrieben aufgearbeitet.
Teil (2) wurde mit 20 g 98 %iger Schwefelsäure versetzt, wonach das Mol-Verhältnis 0,75 : 1 betrug und die Schwefelsäurekonzentration auf 42,8 % - Basis organisch frei - erhöht wurde. Die Mischung wurde anschließend auf 106 °C erhitzt und 2 h unter Rückfluß gehalten. Nach beendeter Reaktion wurde wie im Beispiel 9 beschrieben aufgearbeitet.
Teil (3) wurde mit 40 g 98 %iger Schwefelsäure versetzt, wonach das Molverhältnis Säure zu MMP-CH bzw. MHA-Amid 0,95 : 1 betrug und die Schwefelsäurekonzentration auf 48,5 % - Basis organisch frei - erhöht wurde. Die Mischung wurde anschließend bei 108 - 109 °C 1 h unter Rückfluß gehalten. Danach wurde wie im Beispiel 9 beschrieben aufgearbeitet. Die Ergebnisse sind der folgenden Tabelle zu entnehmen.

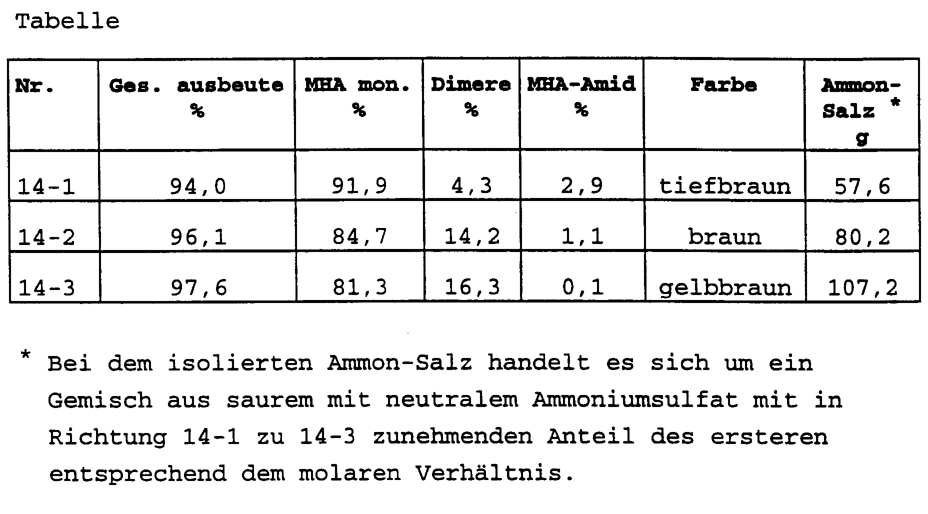

### Beispiel 15:

In einem Reaktionsgefäß wurde 1 Mol MMP-CH vorgelegt. Zwischen 25 - 30 °C dosierte man im Verlauf von 60 Minuten 89 g 75 %ige Schwefelsäure (0,68 Mol) unter guter Durchmischung und Kühlung zu. Nach weiteren 60 Minuten Rühren bei 50 °C war das Cyanhydrin vollständig umgesetzt und analytisch nicht mehr nachweisbar. Das Hydrolysezwischenprodukt wurde mit 180 g Wasser verdünnt, bis zum Rückflußsieden erhitzt und bei 108 °C 3 h hydrolysiert. Die ausreagierte Lösung wurde am Rotationsverdampfer i. V. vollständig eingedampft und der verbleibende Rückstand mit 200 g Aceton digeriert. Nach Filtration der erhaltenen Suspension, Auswaschung des Filterrückstandes mit 2 mal 25 g Aceton, Eindampfung des Filtrates erhielt man 150 g MHA-Endprodukt mit einem Dimerenanteil von 6,5 %, welches mit Wasser zu einer 87 %igen braun gefärbten Lösung verdünnt wurde. Gleichzeitig wurde nach dem Trocknen 77 g ein rein weißes Salzgemisch aus Ammoniumbisulfat + Ammoniumsulfat (98,3 %) erhalten.

### Beispiel 16:

Es wurde eine Hydrolyselösung wie in Beispiel 2 beschrieben hergestellt und entgeistet. Nach der Zugabe von 68 g 25 %igen Ammoniak erhielt man 443 g eines Produktgemisches, welches folgende Zusammensetzung besaß: 33,5 % MHA mit 7,5 % Dimeren, 29,3 % Ammoniumsulfat und 37,1 % Wasser. Dieses Gemisch wurde bei 90 °C unter Vakuum bis zu einem Gewicht von ca. 300 g eingeengt, wonach sich der Dimerengehalt nur unwesentlich auf 7,6 % erhöht hatte. Danach digerierte man die Mischung mit 290 g Methylisobutylketon (MIBK) und destillierte das Restwasser (∼ 21 g) bei 88 °C azeotrop ab. Die resultierende Suspension wurde analog Beispiel 2 weiter aufgearbeitet. Man erhielt 148,7 g MHA mit einem Gehalt an 8,9 % Dimeren und weniger als 1 % Trimeren sowie 131 g rein weißes Ammoniumsulfat. Nach Verdünnen des MHA auf eine Konzentration von 88 % erhielt man eine fahlgelbe Lösung von hervorragender Fließfähigkeit und guter thermischer Stabilität.

### Beispiel 17:

In einer Rührwerkapparatur wurden 10 Mol MMP-CH mit der gleichen Molzahl Schwefelsäure gemäß der in Beispiel 2 beschriebenen Verfahrensweise umgesetzt. Das Roh-Hydrolysat wurde unter Entfernung von 80 g flüchtiger Anteile mittels Vakuum-Verdampfungskühlung bis ca. 65 °C entgeistet und anschließend mit 10 Mol 25 %igen Ammoniak neutralisiert. Die resultierende Lösung wurde nun kontinuierlich in einen beheizten unter Vakuum stehenden Sambay-Verdampfer eingespeist, an welchem kopfseitig Kondensatkühler und Destillatvorlage sowie ablaufseitig zwei gekühlte, mit Rührern versehene Wechselvorlagen angeschlossen waren. Die Entwässerung erfolgte bei 90 °C / 80 mbar, wobei die Produktaufgabe so einreguliert wurde, daß das ablaufende Konzentrat nur noch einen minimalen Restwassergehalt von weniger als 1 % aufwies, welcher laufend mittels Karl-Fischer-Titration kontrolliert wurde. Das Sumpfprodukt wurde alternierend in die mit aliquoten Mengen Aceton beschickten Wechselvorlagen bis auf jeweils gleiche Füllhöhe abgelassen und die entstandenen Suspensionen noch rührfähig blieben. Die einzelnen Fraktionen wurden nach dem Entspannen in einer Labor-Schälzentrifuge getrennt, die Zentrifugate wurden mit wenig Aceton nachgewaschen und nach der Vereinigung getrocknet. Die in einen Sammelbehälter ablaufenden Zentrifugenfiltrate wurden kontinuierlich in einen mit Wechselvorlagen und Kondensationseinrichtungen ausgerüsteten Fallfilmverdampfer eingespeist und unter Vakuum lösungsmittelfrei entgeistet. Das ablaufende Konzentrat wurde alternierend in die mit anteiligen Mengen Wasser beschickten Sumpfvorlagen unter Rührung und Kühlung aufgenommen und bis zu einem Gehalt um 88 % Endprodukt angereichert. Die einzelnen Fraktionen wurden in einem Vorratsbehälter vereinigt und abschließend homogenisiert. Nach Beendigung der Produktaufarbeitung wurden 98,8 % MHA mit einem Gehalt von 11,5 % Dimeren, 1,8 % Trimeren und 0,4 % Ammoniumsulfat als 88 %ige gelbe Lösung erhalten. Die Ausbeute an kristallinem Ammoniumsulfat betrug 95,5 %.

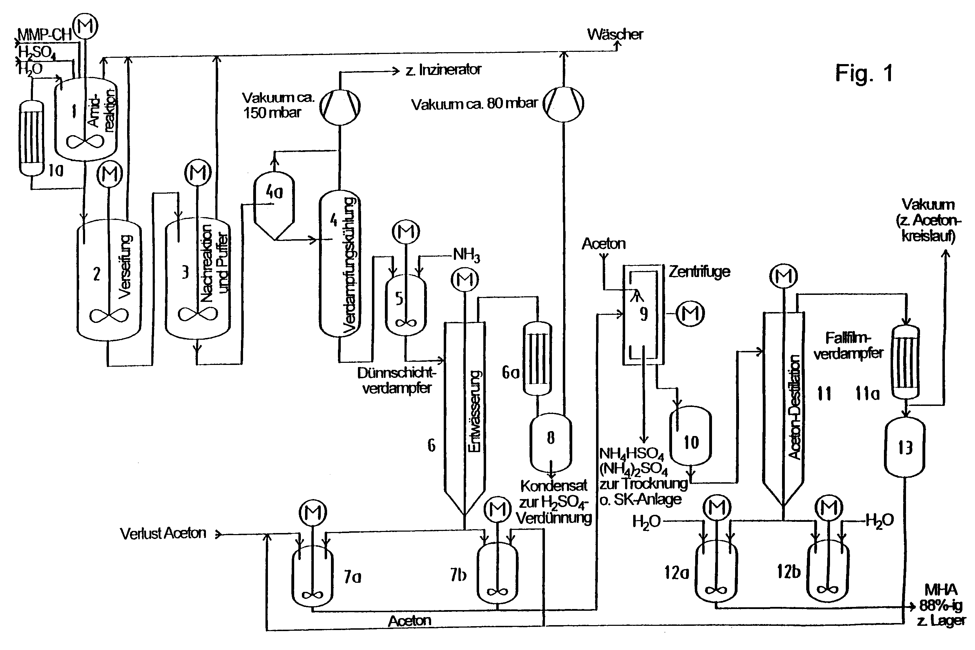

Die im vorliegenden Beispiel beschriebene Herstellung von MHA und Ammoniumsulfat bzw. -bisulfat ist in Figur 1 als technisches Verfahren mit den Hauptapparaten schematisch wiedergegeben.

Die in Figur 1 schematisch mit den Hauptapparaten dargestellte technische Vorrichtung wird in einem Verfahren gemäß der in Beispiel 2 und 17 beschriebenen Arbeitsweise mit ansatzweiser Herstellung und kontinuierlicher Aufarbeitung der Zielprodukte folgendermaßen betrieben:

In einem ersten Rührwerksreaktor 1 wird eine ca. 65 %ige Schwefelsäure bereitet und vorgelegt, worauf durch Zugabe des MMP-Cyanhydrins im stöchiometrisch gleichen Verhältnis oder auch mit geringem Unterschuß der Mineralsäure bei ca. 50 °C die Umsetzung zum MHA-Amid erfolgt, während man die Reaktionswärme über einen äußeren Kühlkreislauf 1a abführt. Nachdem innerhalb einer Nachreaktionszeit das Cyanhydrin vollständig konvertiert ist, läßt man die Mischung in einen zweiten Rührwerksreaktor 2 ab, worauf nach Verdünnung auf eine ca. 40 %ige Schwefelsäurekonzentration unter Temperaturerhöhung bei ca. 95 °C die Hydrolyse des MHA-Amids zur Säure MHA im wesentlichen abgeschlossen wird. Danach überführt man die Mischung in einen dritten Rührwerksbehälter 3, der sowohl als Nachreaktor als auch als Puffer dient. Von dort speist man die Mischung kontinuierlich in eine unter Vakuum stehende Behälterkombination, bestehend aus dem Abscheider 4a und dem Expansionstank 4, ein, wobei die Mischung auf ca. 70 °C abgekühlt wird, während gleichzeitig flüchtige Verunreinigungen und eine anteilige Menge Wasser abgezogen werden, die gasförmig oder kondensiert einem Inzinerator zugeführt werden. Nach der Entspannung gelangt die entgeistete Mischung in einen weiteren Rührwerksbehälter 5, in welchem kontinuierlich durch pH-kontrollierte Zugabe von Ammoniak bis zur vollständigen Neutralisation der Sulfationen unter Bildung von Ammoniumsulfat ein Konzentrat erhalten wird, das man in einen mit Rotor versehenen, unter Vakuum stehenden Dünnschichtverdampfer 6 mit dem Kondensationssystem 6a und 8 einspeist. Bei ca. 90 °C wird das Konzentrat im Vakuum unter Schnellverdampfung soweit entwässert, daß das in Form eines Gels ausgetragene Sumpfprodukt nur noch geringe Restwassergehalte von ca. ≤ 5 % bezogen auf MHA + Oligomere aufweist. Das Sumpfprodukt wird nun unter Entspannung alternierend in den mit dem Lösungsmittel, z. B. Aceton, beschickten Wechselvorlagen 7a und 7b abgelassen, wobei das Ammoniumsulfat auskristallisiert, während das MHA in Lösung geht. Das Verhältnis Produkt zu Lösungsmittel wird so gewählt, daß noch rührfähige Suspensionen entstehen. Im Fall des Acetons ist ein Verhältnis von 1 : 1,5 - 2,0 ausreichend. Die aliquoten Suspensionsfraktionen werden intermittierend einer Schälzentrifuge 9 zugeführt und getrennt sowie mit dem Lösungsmittel nachgewaschen.

Die resultierenden Filterkuchen-Fraktionen werden vereinigt und ggf. zwecks Rückgewinnung noch anhaftender Lösungsmittelreste einer ausdampfenden Nachbehandlung zugeführt (im Schema nicht eingezeichnet). Das in einen Sammelbehälter 10 ablaufende, aus Mutterlauge und Waschlösung bestehende Filtrat wird kontinuierlich in einen unter Vakuum stehenden, mit entsprechender Peripherie ausgestatteten Dünnschicht- oder Fallfilmverdampfer eingespeist und bei 80 - 90 °C unter Schnellverdampfung vollständig vom Lösungsmittel befreit - ggf. unter rektifizierenden Bedingungen, um eine Wasseranreicherung zu verhindern. Das abgezogene Lösungsmittel wird nach der Verflüssigung im Kondensator 11a einem Sammelbehälter 13 zugeführt und nach entsprechender Verlustergänzung wieder in den Kreislaufprozeß zurückgegeben. Das unter Entspannung aus dem Verdampfer ausgetragene, nahezu bis vollständig wasserfreie Sumpfprodukt wird alternierend unter Kühlung in den beiden mit anteiligen Mengen Wasser beschickten Rührwerksvorlagen 12a und 12b aufgenommen, jeweils bis zu einem Endproduktgehalt um 88 - 90 % angereichert und danach ins Lager überführt. Das in der Entwässerungsstufe abgedampfte, in einem Kondensator 8 verflüssigte und in einem Sammelbehälter 8 aufgefangene wässrige Brüdenkondensat kann zur Verdünnung der Schwefelsäure in der ersten wie auch in der zweiten Reaktionsstufe des Prozesses verwendet werden, wodurch das Abwasser bis auf unbedeutende, aus den Gaswäschern herrührende Restmengen reduziert wird.

### Beispiel 18:

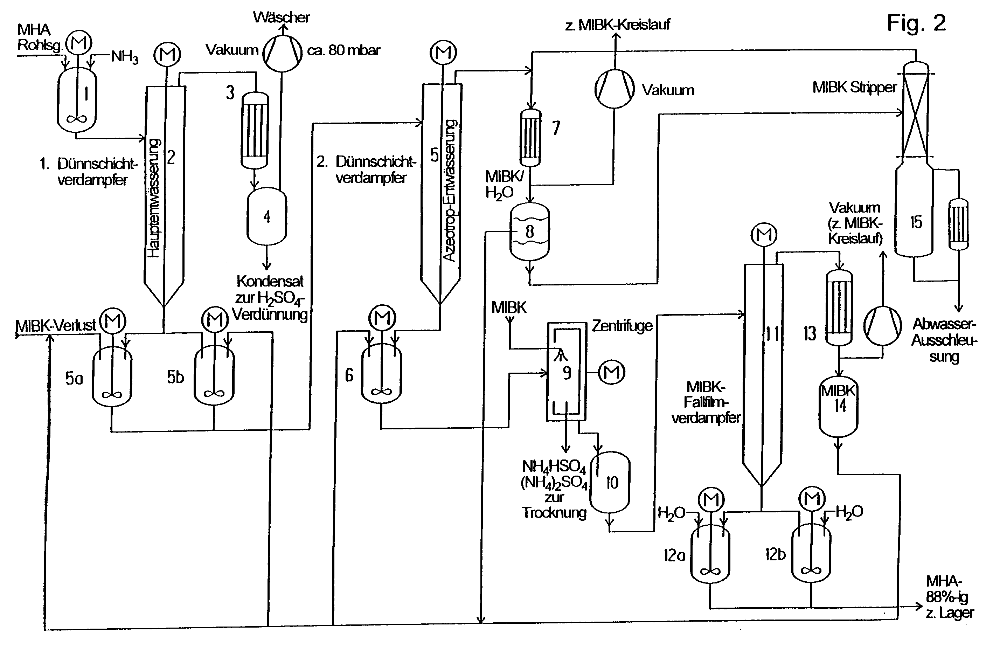

Aus je 10 Mol MMP-CH wurden weitere Hydrolysebatches nach dem allgemeinen Verfahren von Beispiel 2 hergestellt und mittels Verdampfungskühlung bis 65 °C entgeistet. Die Endproduktgewinnung erfolgte jedoch abweichend von der in Beispiel 17 beschriebenen Methode mit Methylisobutylketon (MIBK) zur Produktauftrennung unter Anwendung des in Figur 2 illustrierten Verfahrensschemas:

Die im Rührwerkbehälter 1 mit 25 %iger Ammoniaklösung neutralisierten und dabei auf ca. 70 °C erwärmten Batches (Gewicht: 4350 - 4550 g, Zusammensetzung: 32 - 34 % MHA, 29 - 30,5 % Ammoniumsulfat, Rest Wasser) wurden kontinuierlich in einen 1. Dünnschichtverdampfer 2 eingespeist und durch Schnellverdampfung bei 90 °C / 80 mbar aufkonzentriert, wobei ca. 80 - 90 % des insgesamt vorhandenen Wassers ausgetrieben und über den Kondensator 3 in der Vorlage 4 aufgefangen wurde. Die noch Restwasser enthaltenden Konzentrate wurden alternierend in den mit MIBK im Gewichtsverhältnis 1 : 2 beschickten Vorlagen 5a, 5b unter Rührung und Kühlung aufgenommen. Die resultierenden Suspensionsfraktionen wurden kontinuierlich einem zweiten Dünnschichtverdampfer (Sambay) 5 aufgegeben und bei 70 - 75 °C im Vakuum azeotrop entwässert. Das ablaufende gelartige Produktgemisch wurde in der gekühlten Vorlage 6 in MIBK unter stetiger Ergänzung der Verdampfungsverluste ausgerührt. Die resultierende Suspension wurde mit Hilfe eines geeigneten Förderorgans portionsweise ausgetragen und auf die Schälzentrifuge 9 gegeben. Die abzentrifugierten und mit MIBK gewaschenen Kristallfraktionen wurden in einer Vorratstrommel zwecks späterer Trocknung gesammelt. Die im Filtratbehälter 10 vereinigten Filtrate wurden kontinuierlich in den Fallfilmverdampfer 11 eingespeist und im Vakuum lösungsmittelfrei destilliert. Das ablaufende Sumpfprodukt wurde alternierend in den Vorlagen 12a, 12b aufgenommen und mit Wasser bis zu einer Konzentration um 88 % verdünnt. Die konditionierten Fraktionen wurden in einem Vorratsbehälter zusammengeführt und bilanziert. Die Ausbeuten an MHA und Ammoniumsulfat lagen um 99 % bei einem Dimerengehalt des MHA von unter 10 %. Die MHA-Lösung war von fahlgelber Färbung, guter Fließfähigkeit und thermischer Stabilität. Das bei der Azeotrop-Entwässerung in dem Phasentrenngefäß 8 abgeschiedene MIBK wurde mit dem über die Brüdenkondensation 13, 14 zurückgewonnenen Hauptstrom vereinigt und in einen erneuten Aufarbeitungszyklus, jetzt aber als mit Wasser gesättigtes MIBK (∼ 2 % H₂O) zurückgeführt.

Die im Phasentrenngefäß 8 akkummulierten wässrigen Phasen wurden zur Entfernung von restlichem MIBK in der Strippkolonne 15 unter Vakuum andestilliert. Das wiederum azeotrop übergehende MIBK wurde in das mit der Kolonne 15 kommunizierende Kondensationssystem 7 und 8 des Dünnschichtverdampfers 5 zurückgeführt.

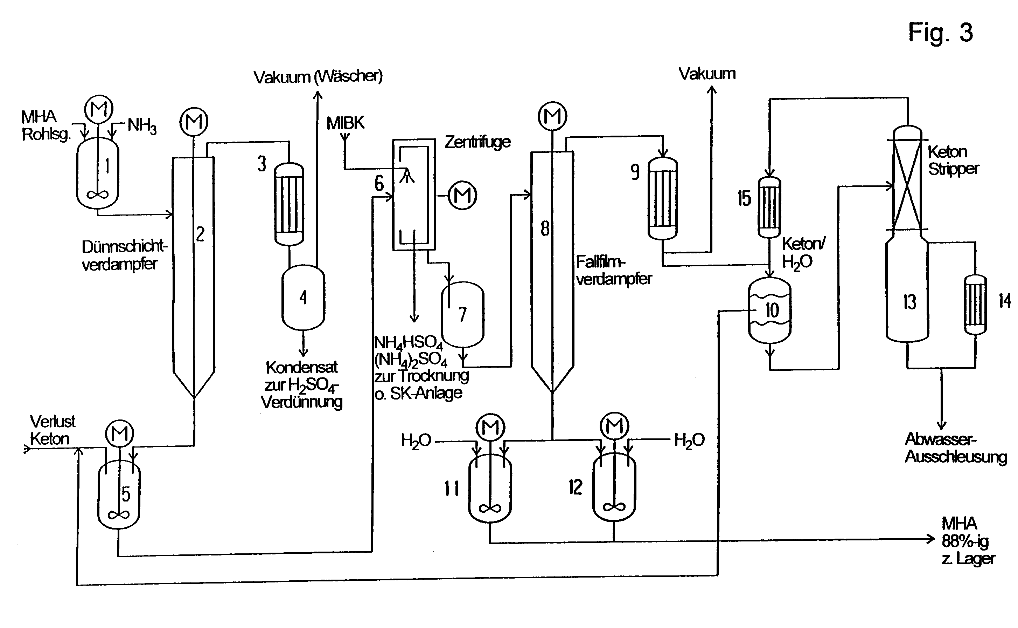

In Figur 3 ist eine vereinfachte technische Durchführung des erfindungsgemäßen Verfahrens mit einem beliebigen Lösungsmittel wiedergegeben. Die Bedeutung der verwendeten Bezugszeichen entspricht den in den Figuren 1 und 2 verwendeten Bezugszeichen.

### Beispiel 19:

Es wurde wie in Beispiel 18 verfahren, jedoch wurden zur Produkttrennung nacheinander die folgenden Lösungsmittel verwendet: 1. Methylisopropylketon - 2. Ethyl-n-amylketon - 3. Essigsäureisobutylazetat. Ausbeuten und Produkteigenschaften unterschieden sich nicht wesentlich vom vorhergehenden Beispiel.

### Beispiel 20:

Es wurde wie in Beispiel 18 verfahren, jedoch wurde zur Auftrennung des Salz/MHA-Gemisches Methyl-t-butylether verwendet und die Lösungsmitteldestillationen in den Verdampfungsstufen 5, 11, 15 von Figur 2 wurden unter Normaldruck durchgeführt. Die Ergebnisse waren denen von Beispiel 18 vergleichbar.

### Beispiel 21:

In einem emaillierten, doppelt ummantelten Rührwerksreaktor mit Rückflußkühler nebst Abgaswäscher mit angeschlossener Vakuumpumpe wurden 150 l demineralisiertes Wasser vorgelegt und 300 kg 98 %ige Schwefelsäure (3,0 Kmol) eingemischt. Anschließend dosierte man unter intensiver Durchmischung 408 kg 96,5 %iges MMP-CH so zu, daß nach Erreichen der Reaktionstemperatur von 50 - 55 °C die Reaktionswärme über den mit Kühlsole beaufschlagten Doppelmantel abgeführt werden konnte. Nach beendetem Zulauf ließ man noch 30 Minuten nachreagieren und kontrollierte auf vollständigen Umsatz des Cyanhydrins. Die Hydrolysezwischenprodukt-Mischung, die im wesentlichen aus dem Säureamid MHA-Amid bestand, wurde mit 360 l demineralisiertem Wasser verdünnt, auf 90 °C erhitzt und 3 h bei dieser Temperatur gerührt. Danach war die Hydrolyse vollständig und MHA-Amid nicht mehr nachweisbar. Nachdem man die Kühlung entfernt hatte, wurde Vakuum angelegt und im Verlauf von ca. 30 Minuten die Temperatur mittels Verdampfungskühlung auf ca. 65 °C abgesenkt während gleichzeitig geringe Mengen an flüchtigen Organika neben anteiligen Mengen Wasser, zusammen ca. 36 kg abgetrieben und im Abgaswäscher absorbiert wurden.

Die Absorptionsflüssigkeit wurde mit Wasserstoffperoxid bei pH 9 entgiftet sowie desodoriert und anschließend an das Abwassernetz abgegeben. Nach der Entgeistung erhielt man 1172 kg (960 l) Hydrolyselösung mit der Zusammensetzung 37,9 % MHA (mit Oligomeren), 29,4 % Ammoniumbisulfat und 32,5 % Wasser. Die Lösung wurde in eine auf 65 °C temperierte Meßvorlage eingepumpt und aus dieser kontinuierlich in einen dampfbeheizten Dünnschichtverdampfer eingesaugt, der mit einem starrflügeligen Rotor ausgestattet war, eine Heizfläche von 1,0 m² aufwies und über einen Brüdenkondensator nebst Vorlage an eine Vakuumanlage angeschlossen war. Mit größtmöglicher Durchsatzgeschwindigkeit wurde die Lösung bei 90 °C / 80 mbar bis auf weniger als 0,5 % Wasser im Sumpfprodukt entwässert. Das ablaufende Konzentrat wurde unter Entspannung mittels einer Drehkolbenpumpe in eine mit Aceton zur Hälfte gefüllten sowie gekühlten Rührvorlage eingepumpt, wobei sich das Salz in kristalliner Form abschied. Nach entsprechender Feststoffanreicherung wurde die gebildete Suspension portionsweise auf eine Pendelzentrifuge gegeben während gleichzeitig die ausgetragene Acetonmenge in der niveaugeregelten Rührvorlage wieder ergänzt wurde. Das von der Zentrifuge in einen Sammelbehälter ablaufende Filtrat wurde kontinuierlich in einen warmwasserbeheizten Fallfilmverdampfer von 0,5 m² Heizfläche eingespeist, der über die gleichen Zusatzaggregate wie der Dünnschichtverdampfer verfügte, und bei 80 °C / 160 mbar lösungsmittelfrei ausdestilliert. Das ablaufende Konzentrat wurde unter Entspannung mit einer Drehkolbenpumpe in eine gekühlte Rührvorlage abgepumpt, in welcher bereits die für den gesamten Ansatz berechnete Wassermenge zur Einstellung einer Endkonzentration von 88 % MHA vorgelegt worden war. Nach der jeweils letzten Beaufschlagung und Zentrifugation einer Zentrifugenfüllung wurde der Filterkuchen wiederholt mit Aceton ausgewaschen, geschleudert und in Vorratstrommeln zwecks späterer Trocknung gesammelt. Das in den mit Sole gekühlten Kondensatoren der Destillation sowie der in einem Schaufeltrockner vorgenommenen Salztrocknung zurückgewonnene Aceton mit weniger als 0,2 % Wasser konnte ohne Rektifikation nach Verlustergänzung wieder eingesetzt werden.

Nach Beendigung des Aufarbeitungszyklus wurden 442 kg (98,1 %) Gesamt-MHA mit 13,8 % Dimeren, 2,6 % Trimeren sowie 0,2 % Sulfat als 88 %ige gelb-bräunliche Lösung erhalten. Außerdem wurden 342 kg (99 %) Ammoniumbisulfat isoliert. Durch thermische Spaltung in einer Schwefelsäure-Kontaktanlage können hieraus ca. 285 - 290 kg Schwefelsäure (100 %ig) zurückgewonnen werden.

Die in Beispiel 21 im halbtechnischen Maßstab beschriebene Arbeitsweise steht beispielhaft für ein großtechnisches Verfahren mit ansatzweiser (batchweiser) Herstellung und kontinuierlicher Aufarbeitung mit einem beliebigen Lösungsmittel (bevorzugt Keton), wobei ohne vorherige Neutralisation das resultierende Ammoniumbisulfat in einer angeschlossenen Schwefelsäure-Kontaktanlage zwecks Rückgewinnung von Schwefelsäure und Bildung von Stickstoff verwertet wird.

## Patentansprüche

1. Verfahren zur Herstellung von 2-Hydroxy-4-methylthiobuttersäure (MHA), bei dem das MHA aus einem Reaktionsgemisch isoliert wird, welches durch Anlagerung von Blausäure (HCN) an Methylmercaptopropionaldehyd (MMP) und Hydrolyse des dabei erhaltenen Methylmercaptopropionaldehyd-Cyanhydrins (MMP-CH) mit Schwefelsäure erhalten wird,
**dadurch gekennzeichnet,**
daß das Reaktionsgemisch unter Erhalt eines geringen Restwassergehalt aufweisenden bis praktisch Restwasserfreien MHA-haltigen Salzrückstandes eingedampft wird,
der MHA-haltige Salzrückstand anschließend mit einem organischen Lösungsmittel unter Erhalt einer Suspension behandelt wird,
anschließend die festen Bestandteile aus der Suspension unter Erhalt einer MHA-haltigen Lösung abgetrennt werden,
danach das organische Lösungsmittel aus der MHA-haltigen Lösung unter Erhalt eines MHA-Rückstands entfernt wird
und ggf. danach der MHA-Rückstand durch Zusatz von Wasser konditioniert wird.

2. Verfahren nach Anspruch 1,
**dadurch gekennzeichnet,**
daß das Reaktionsgemisch auf einen Restwassergehalt von ≤ 5 % bezogen auf MHA + Oligomere eingedampft wird.

3. Verfahren nach Anspruch 1,
**dadurch gekennzeichnet,**
daß das Reaktionsgemisch im wesentlichen vollständig von Wasser befreit wird.

4. Verfahren nach einem der Ansprüche 1 bis 3,
**dadurch gekennzeichnet,**
daß die Eindampfung des Reaktionsgemisches kontinuierlich ausgeführt wird.

5. Verfahren nach einem der Ansprüche 1 bis 4,
**dadurch gekennzeichnet,**
daß das Reaktionsgemisch vor und/oder während der Eindampfung einer adiabatischen Verdampfungskühlung unter Anlegen eines Vakuums bis auf ca. 60 °C oder weniger unterzogen wird, um flüchtige und/oder geruchsintensive Bestandteile im Reaktionsgemisch zu entfernen.

6. Verfahren nach einem der Ansprüche 1 bis 5,
**dadurch gekennzeichnet,**
daß die Eindampfung des Reaktionsgemisches nach vorheriger Neutralisation mit Ammoniak erfolgt.

7. Verfahren nach einem der vorhergehenden Ansprüche,
**dadurch gekennzeichnet,**
daß der MHA-haltige Salzrückstand nach der weitgehend vollständigen Befreiung von Wasser als Sumpfprodukt der Eindampfung mit Aceton, Methylisopropylketon, Methylisobutylketon, Isopropanol, Toluol oder Tetrahydrofuran als organischem Lösungsmittel aufgenommen wird.

8. Verfahren nach einem der vorhergehenden Ansprüche,
**dadurch gekennzeichnet,**
daß die Suspension filtriert wird, wobei als feste Bestandteile kristalline Ammoniumsulfat-Salze in einer Reinheit ≥ 99 % und die MHA-haltige Lösung anfallen.

9. Verfahren nach einem der vorhergehenden Ansprüche,
**dadurch gekennzeichnet,**
daß das Lösungsmittel aus der MHA-haltigen Lösung unter rektifizierenden und/oder azeotropbildenden Bedingungen abgetrennt wird, wobei das Lösungsmittel, welches zurückgewonnen wird, weniger als 5 % Wasser enthält.

10. Verfahren nach einem der vorhergehenden Ansprüche,
**dadurch gekennzeichnet,**
daß die Hydrolyse des MMP-CH in zwei Stufen ausgeführt wird, wobei in einer ersten Stufe MHA-Amid und in einer zweiten Stufe MHA erhalten werden.

11. Verfahren nach Anspruch 10,
**dadurch gekennzeichnet,**
daß MMP-CH in der ersten Stufe mit 60 - 85 %iger, vorzugsweise 65 - 75 %iger, Schwefelsäure im Molverhältnis 1 : 0,5 bis 1 : 1,0, vorzugsweise 1 : 0,55 bis 1 : 0,95, bei Temperaturen zwischen 20 und 60 °C, vorzugsweise 30 - 50 °C, hydrolysiert wird.

12. Verfahren nach Anspruch 11,
**dadurch gekennzeichnet,**
daß das in der ersten Hydrolysestufe erhaltene Gemisch frei von nicht umgesetzten Cyanhydrin ist.

13. Verfahren nach einem der Ansprüche 10 bis 12,
**dadurch gekennzeichnet,**
daß das MHA-Amid in der zweiten Stufe unter Zugabe von Wasser und geringstenfalls weiterer Schwefelsäure bis zur stöchiometrischen Obergrenze bei Temperaturen von 90 - 110 °C oder unter Rückflußbedingungen hydrolysiert wird.

14. Verfahren zur Herstellung von 2-Hydroxy-4-methylthiobuttersäure (MHA), bei dem das MHA aus einem Reaktionsgemisch isoliert wird, welches durch Anlagerung von Blausäure (HCN) an Methylmercaptopropionaldehyd (MMP) und Hydrolyse des dabei erhaltenen Methylmercaptopropionaldehyd-Cyanhydrins (MMP-CH) mit Schwefelsäure erhalten wird,
**dadurch gekennzeichnet,**
daß die Isolierung des MHA aus dem Reaktionsgemisch eine Fest/Flüssig-Trennung aufweist, bei der ein im wesentlichen gallertartiger oder fester MHA-haltiger Salzrückstand mit einem organischen Lösungsmittel behandelt wird.

15. Verfahren nach Anspruch 8,
**dadurch gekennzeichnet,**
daß man die in fester Form erhaltenen Ammoniumsulfat-Salze ggf. nach vorheriger Ausdampfung von Lösungsmittelresten und Anmaischen mit Wasser einer Schwefelsäure-Kontaktanlage unter Rückgewinnung von Schwefelsäure zuführt.

16. Verfahren nach einem der Ansprüche 1 - 5 oder 8, sofern dieser nicht auf Anspruch 6 rückbezogen ist,
**dadurch gekennzeichnet,**
daß die Eindampfung des Reaktionsgemisches ohne vorherige oder nachfolgende Neutralisation mit Ammoniak durchgeführt wird, und daß die in fester Form erhaltenen Ammoniumsulfat-Salze ggf. nach vorheriger Ausdampfung von Lösungsmittelresten und Anmaischen mit Wasser einer Schwefelsäure-Kontaktanlage unter Rückgewinnung von Schwefelsäure zugeführt wird.

## Claims

1. Process for the preparation of 2-hydroxy-4-methylthiobutyric acid (MHA), wherein MHA is isolated from a reaction mixture which is obtained by addition of hydrocyanic acid (HCN) to methylmercaptopropionaldehyde (MMP) and hydrolysis with sulfuric acid of the methylmercaptopropionaldehyde cyanohydrin (MMP-CH) thus obtained,
characterised in that
the reaction mixture is evaporated, an MHA-containing salt residue having a low residual water content to virtually no residual water content being obtained,
the MHA-containing salt residue is then treated with an organic solvent, a suspension being obtained,
the solid constituents are then separated from the suspension, an MHA-containing solution being obtained,
thereafter the organic solvent is removed from the MMA-containing solution, an MHA residue being obtained
and thereafter the MHA residue is optionally conditioned by addition of water.

2. Process according to claim 1,
characterised in that
the reaction mixture is evaporated to a residual water content of ≤ 5%, based on MHA + oligomers.

3. Process according to claim 1,
characterised in that
the reaction mixture is substantially completely freed from water.

4. Process according to one of claims 1 to 3,
characterised in that
the evaporation of the reaction mixture is conducted continuously.

5. Process according to one of claims 1 to 4,
characterised in that
before and/or during the evaporation the reaction mixture is subjected to an adiabatic evaporative cooling, with application of a vacuum, down to approximately 60°C or less, in order to remove volatile and/or highly odorous constituents in the reaction mixture.

6. Process according to one of claims 1 to 5,
characterised in that
the evaporation of the reaction mixture is carried out after previous neutralisation with ammonia.

7. Process according to one of the preceding claims,
characterised in that
the MHA-containing salt residue constituting the bottom product of the evaporation, after the largely complete liberation of water, is taken up by acetone, methyl isopropyl ketone, methyl isobutyl ketone, isopropanol, toluene or tetrahydrofuran as organic solvent.

8. Process according to one of the preceding claims,
characterised in that
the suspension is filtered, with the recovery of crystalline ammonium sulfate salts in a purity of ≥ 99% as solid constituents and of the MHA-containing solution.

9. Process according to one of the preceding claims,
characterised in that
the solvent is separated from the MHA-containing solution under rectifying and/or azeotrope-forming conditions, the solvent which is recovered containing less than 5% water.

10. Process according to one of the preceding claims,
characterised in that
the hydrolysis of the MMP-CH is carried out in two steps, MHA amide being obtained in a first step and MHA being obtained in a second step.

11. Process according to claim 10,
characterised in that
in the first step MMP-CH is hydrolysed with 60 - 85% and preferably 65 - 75% sulfuric acid in the molar ratio of 1 : 0.5 to 1 : 1.0 and preferably of 1 : 0.55 to 1 : 0.95, at temperatures of between 20°C and 60°C and preferably of 30°C to 50°C.

12. Process according to claim 11,
characterised in that
the mixture obtained in the first hydrolysis step is free from unreacted cyanohydrin.

13. Process according to one of claims 10 to 12,
characterised in that
in the second step the MHA amide is hydrolysed with addition of water and at the very least additional sulfuric acid up to the stoichiometric upper limit, at temperatures of 90°C to 110°C or under reflux conditions.

14. Process for the preparation of 2-hydroxy-4-methylthiobutyric acid (MHA), wherein MHA is isolated from a reaction mixture which is obtained by addition of hydrocyanic acid (HCN) to methylmercaptopropionaldehyde (MMP) and hydrolysis with sulfuric acid of the methylmercaptopropionaldehyde cyanohydrin (MMP-CH) thus obtained,
characterised in that
the isolation of the MHA from the reaction mixture includes a liquid/liquid separation, wherein a substantially gelatinous or solid MHA-containing salt residue is treated with an organic solvent.

15. Process according to claim 8,
characterised in that
the ammonium sulfate salts obtained in solid form, optionally after previous evaporation of residual solvent and slurrying with water, are passed to a sulfuric acid contact plant, with recovery of sulfuric acid.

16. Process according to one of claims 1 to 5 or 8, unless the latter is related back to claim 6,
characterised in that
the evaporation of the reaction mixture is carried out without previous or subsequent neutralisation with ammonia, and that the ammonium sulfate salts obtained in solid form, optionally after previous evaporation of residual solvent and slurrying with water, are passed to a sulfuric acid contact plant, with recovery of sulfuric acid.

## Revendications

1. Procédé pour la préparation de l'acide 2-hydroxy-4-méthylthiobutyrique (MHA), dans lequel on isole le MHA d'un mélange réactionnel que l'on obtient via fixation par addition d'acide cyanhydrique (HCN) sur du méthylmercaptopropionaldéhyde (MMP) et par hydrolyse de la méthylmercaptopropionaldéhydecyanhydrine (MMP-CH) obtenue en l'occurrence, avec de l'acide sulfurique,
caractérisé en ce que
on évapore le mélange réactionnel pour obtenir un résidu salin contenant du MHA dont la teneur en eau résiduelle s'étend d'une valeur minime jusqu'à une valeur pratiquement nulle,
on traite ensuite le résidu salin contenant du MHA avec un solvant organique pour obtenir une suspension,
on sépare ensuite les constituants solides de la suspension pour obtenir une solution contenant du MHA,
on élimine ensuite le solvant organique de la solution contenant du MHA pour obtenir un résidu de MHA,
et on conditionne le cas échéant par la suite le résidu de MHA par addition d'eau.

2. Procédé selon la revendication 1,
caractérisé en ce que
on évapore le mélange réactionnel jusqu'à ce que l'on obtienne une teneur en eau résiduelle ≤ 5% rapportés au MHA + des oligomères.

3. Procédé selon la revendication 1,
caractérisé en ce que
on libère l'eau du mélange réactionnel de manière essentiellement complète.

4. Procédé selon l'une quelconque des revendications 1 à 3,
caractérisé en ce que
on effectue l'évaporation du mélange réactionnel en continu.

5. Procédé selon l'une quelconque des revendications 1 à 4,
caractérisé en ce que
on soumet le mélange réactionnel avant et/ou pendant l'évaporation, à un refroidissement adiabatique par évaporation en appliquant un vide, jusqu'à une température d'environ 60°C ou moins pour éliminer les constituants volatils et/ou les constituants dégageant une odeur prononcée dans le mélange réactionnel.

6. Procédé selon l'une quelconque des revendications 1 à 5,
caractérisé en ce que
l'évaporation du mélange réactionnel a lieu après neutralisation préalable avec de l'ammoniac.

7. Procédé selon l'une quelconque des revendications précédentes,
caractérisé en ce que
on reprend le résidu salin contenant du MHA, après la libération largement complète de l'eau sous forme de produit de bas de colonne issu de l'évaporation, avec de l'acétone, de la méthylisopropylcétone, de la méthylisobutylcétone, de l'isopropanol, du toluène ou du tétrahydrofuranne à titre de solvant organique.

8. Procédé selon l'une quelconque des revendications précédentes,
caractérisé en ce que
on filtre la suspension pour obtenir, à titre de constituants solides, des sels cristallins de sulfate d'ammonium avec une pureté ≥ 99%, ainsi que la solution contenant du MHA.

9. Procédé selon l'une quelconque des revendications précédentes,
caractérisé en ce que
on sépare le solvant de la solution contenant du MHA dans des conditions de rectification et/ou de formation d'azéotrope, le solvant que l'on récupère contenant de l'eau à concurrence de moins de 5%.

10. Procédé selon l'une quelconque des revendications précédentes,
caractérisé en ce qu'on effectue l'hydrolyse de la MMP-CH en deux étapes dans lesquelles on obtient du MHA-amide dans une première étape et du MHA dans une seconde étape.

11. Procédé selon la revendication 10,
caractérisé en ce que
on hydrolyse la MMP-CH dans la première étape avec de l'acide sulfurique à 60-85%, de préférence à 65-75% dans le rapport molaire de 1:0,5 à 1:1,0, de préférence de 1:0,55 à 1:0,95, à des températures entre 20 et 60°C, de préférence de 30 à 50°C.

12. Procédé selon la revendication 11,
caractérisé en ce que
le mélange obtenu dans la première étape d'hydrolyse est exempt de cyanhydrine n'ayant pas réagi.

13. Procédé selon l'une quelconque des revendications 10 à 12,
caractérisé en ce que
on hydrolyse le MHA-amide dans la deuxième étape en ajoutant de l'eau et au minimum une quantité supplémentaire d'acide sulfurique jusqu'à la limite stoechiométrique supérieure à des températures de 90 à 110°C ou dans des conditions de reflux.

14. Procédé pour la préparation de l'acide 2-hydroxy-4-méthylthiobutyrique (MHA), dans lequel on isole le MHA d'un mélange réactionnel que l'on obtient via fixation par addition d'acide cyanhydrique sur du méthylmercaptopropionaldéhyde (MMP) et par hydrolyse de la méthylmercaptopropionaldéhydecyanhydrine (MMP-CH) obtenue en l'occurrence, avec de l'acide sulfurique,
caractérisé en ce que
l'isolation du MHA du mélange réactionnel présente une séparation phase solide/phase liquide, dans laquelle on traite un résidu salin essentiellement gélatineux ou solide contenant du MHA avec un solvant organique.

15. Procédé selon la revendication 8,
caractérisé en ce que
on achemine les sels de sulfate d'ammonium obtenus sous forme solide, éventuellement après avoir soumis les résidus de solvants à une évaporation préalable et à un malaxage avec de l'eau, à une installation de contact avec de l'acide sulfurique, avec récupération de l'acide sulfurique.

16. Procédé selon l'une quelconque des revendications 1 à 5 ou selon la revendication 8 pour autant que cette dernière se rapporte à la revendication 6,
caractérisé en ce que
on effectue l'évaporation du mélange réactionnel sans neutralisation préalable ou ultérieure avec de l'ammoniac et en ce qu'on achemine les sels de sulfate d'ammonium obtenus sous forme solide éventuellement après avoir soumis les résidus de solvants à une évaporation préalable et à un malaxage avec de l'eau, à une installation de contact avec de l'acide sulfurique, avec récupération de l'acide sulfurique.
